# EUROPEAN PATENT SPECIFICATION

(11) **EP 0 613 384 B1**
(45) Date of publication and mention of the grant of the patent: **29.07.1998**
(21) Application number: 92906638.9
(22) Date of filing: 18.03.1992
(51) Int. Cl.: A61M 15/00

(54) **INHALER**
INHALATIONSGERAT
INHALATEUR

(30) Priority: 28.03.1991 GB 9106648
(43) Date of publication of application: 07.09.1994
(73) Proprietor: RHONE-POULENC RORER LIMITED, West Malling, Kent ME19 4TA (GB)
(72) Inventor: COOK, Robert, Stanley, Dagenham Essex RM10 7XS (GB); HOBBS, Michael, Anthony, Dagenham Essex RM10 7XS (GB); LEIGHTON, Ann-Marie, Dagenham Essex RM10 7XS (GB); SIMPKIN, Gordon, Thomas, Dagenham Essex RM10 7XS (GB); TRUNLEY, Roy, Dagenham Essex RM10 7XS (GB)
(74) Representative: Caffin, Lee
(86) International application number: GB9200480
(87) International publication number: WO9217233

(56) References cited:
- EP-A- 0 129 985
- EP-A- 0 147 755
- EP-A- 0 303 844
- EP-A- 0 406 893
- GB-A- 2 061 735
- US-A- 4 069 819

## Description

The present invention relates to an inhaler, according to the preamble of claim 1, for allowing a patient to inhale powdered medicament from a capsule.

Various forms of inhaler are already known, and it is known to provide means for piercing the ends of a capsule in order to allow the medicament to be withdrawn during inhalation.

Extraction of the medicament may occur as a result of the inhaled airstream passing over the capsule causing it to spin by virtue of a vortical configuration of the airstream through the inhaler. This spinning of the capsule assists ejection of the powdered medicament within the tumbling capsule to enter the inhaled airstream.

The means for piercing the medicament capsule are known to comprise pins which can be driven inwardly through the ends of the capsule, thereby puncturing the capsule by virtue of sharpened ends on the piercing pins. It is a disadvantage of the known forms of capsule-piercing inhalers that misuse of the inhaler, for example by a child, or accidental mis-use by any patient may result in piercing of a human finger by operation of the capsule piercing pins.

Since it is customary to open the chamber of the device to allow insertion of a fresh capsule, and since it is also possible for the capsule-piercing pins to be effective inside the chamber, in order to ensure that the pierced capsule remains enclosed in the chamber between piercing and inhalation agitation of the capsule, it is possible for there to be access to the chamber, sufficient to allow a finger to enter the space normally occupied by a capsule being pierced, hence leading to injury.

EP-A-0129985 discloses an inhaler using the medicament in a blister pack which is placed in position on a discharge plate while the inhaler housing is open, and then as the housing is closed a spike on the inside of the cover pierces the blister pack to discharge the contents into a space from which they can be inhaled by the user. Closing the cover effects the spiking action and a careless user could damage his or her fingers during that closing action.

EP-A-0303844 discloses an inhaler using elongate capsules carried by a rotating holder which in one position aligns the ends of the capsule with piercing pins actuable by squeezing the sides of the inhaler housing, and in another position aligns the longitudinal axis of the thus pierced capsule, while held stationary in the carrier, to allow inhalation air to pass directly through the capsule from end to end. There is no free chamber in which the capsule can rotate in swirling inhalation airflow, and this lack of free rotation of the discharging capsule is associated with the fixed holding of the capsule in such a position that the piercing pins can enter the ends of the capsule without any danger of them contacting the fingers of the user. However, the problem of preventing damage in the case of a capsule piercing means used in conjunction with a swirling chamber does not arise in that disclosure.

US-A-4069819 discloses an inhaler chamber having a small clearance between the exterior of an elongate capsule and the walls of the chamber, thereby allowing the already pierced capsule to nutate so as to shake the contents out through one violently oscillating end of the capsule during inhalation. The inhaler has the appearance of a fountain pen having one body part in which the pins are located and which, when squeezed towards the other body part, causes the pins to enter the capsule. However, equally any attempt to squeeze the pin-containing body part between the finger and thumb without connection to the first body part will equally expose the pins to damage the fingers of the user.

It is an object of the present invention to guard against either inadvertent or deliberate mis-use of the inhaler resulting in damage to a finger in the chamber.

Accordingly, the present invention provides an inhaler for inhaling powdered medicament from capsules which are opened in the inhaler, comprising:- a swirling chamber for holding a capsule from which the powdered medicament is extracted by rotation of the opened capsule in the inhalation airflow through the swirling chamber; means in the swirling chamber for opening a capsule having been inserted in the swirling chamber ready for inhalation; a closure member movable between a first position closing the swirling chamber and a second position providing access to the swirling chamber for insertion of a capsule, and interlock means responsive to positioning of the closure member in said second position for inhibiting operation of said closure opening means.

In order that the present invention may more readily be understood, the following description is given, merely by way of example, with reference to the accompanying drawings, in which:-
Figure 1 is a side elevational view of an inhaler in accordance with the present invention, showing a cover over the mouthpiece sectioned to facilitate appreciation of the mouthpiece itself;
Figure 2 is an elevational view taken along the direction of arrow II in Figure 1;
Figure 3 is a sectional view showing on the right-hand side the position of a capsule-piercing pin during the capsule rupturing operation, and on the left-hand side the capsule-piercing pin retracted;
Figure 4 is an elevational view of a removable capsule reservoir used in the device of Figures 1 to 3;
Figure 5 is a sectional view taken on the line V-V of Figure 3; and
Figure 6 shows a detail of a modified form of the inhaler, which is to be assembled by catch engagement.

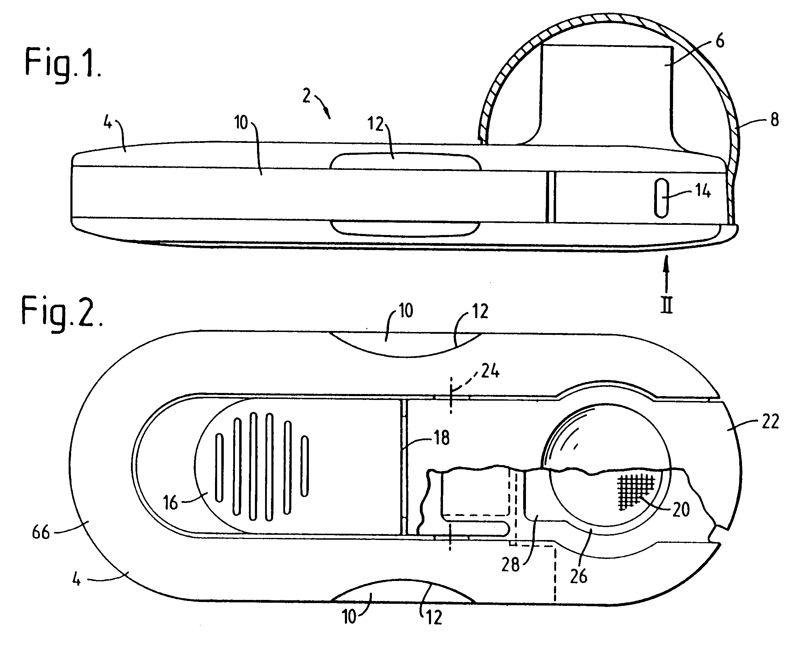

Figure 1 shows the inhaler 2 as comprising a main body 4 having a mouthpiece 6 closed by a cover 8 which is a friction-fit on the inhaler body.

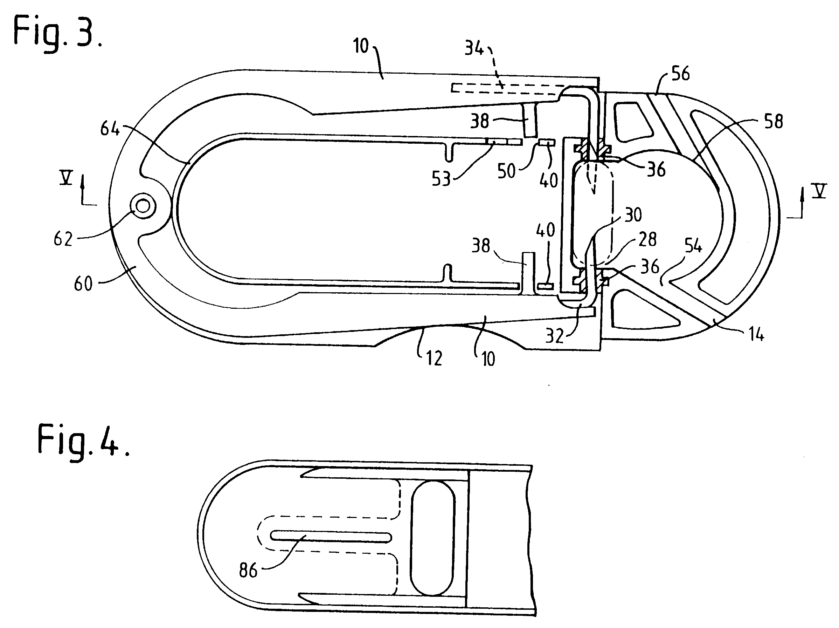

Along each side of the inhaler body 4 there can be seen a limb of a U-shaped spring 10 which can be squeezed towards the other limb by applying pressure between the finger and thumb of the user resting in register with arcuate recesses 12 also shown in Figures 2 and 3.

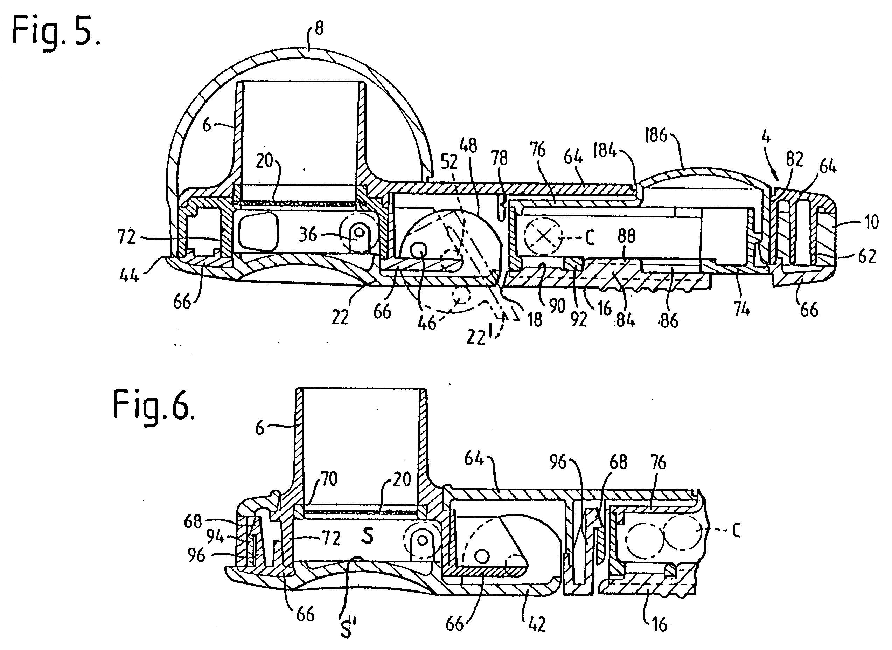

Figure 1 also shows, at the top of the main body 4, an air inlet 14 which forms one of two air inlets to a swirling chamber where the ruptured capsule is subjected to a vortical motion to spin the capsule and to impact it against the walls of the chamber to eject the powdered medicament from the interior of the capsule. In order to enhance the swirling action of the capsule, the chamber has opposed top and bottom walls (see Figs. 5 and 6) giving it a generally flat cylindrical form with the axial dimension slightly greater than the diameter of a capsule but less than the length of a capsule and with a diameter of the chamber in excess of the length of a capsule. The type of capsule which is conventionally used for storing powdered medicament is formed of a capsule body and a capsule cap which fit together with the capsule cap over the capsule body, giving the finished capsule the shape of a generally cylindrical body having hemispherical ends. By "diameter" of the capsule we mean the diameter of the generally cylindrical central portion, and by "length" of the capsule, we mean the total length of the generally cylindrical central portion and its two domed ends.

The front elevational view shown in Figure 2, viewed along the direction of the arrow II of Figure 1, shows the exposed parts of the limbs of the U-shaped spring 10 visible at the arcuate recesses 12 in the main body portion 4.

Figure 2 also shows a sliding cover 16 of an optionally removable capsule reservoir box to be described in more detail with reference to Figures 4 and 5. Sliding the cover 16 downwardly from the position shown in Figure 2 retracts the top edge 18 of the lid to expose one end of the capsule reservoir box interior for release of a capsule from the normally closed reservoir.

Figure 2 also shows, in a partly cut-away region of the inhaler, a screen grid 20 on the wall of the chamber nearer the mouthpiece 6, in order to avoid fragments of the capsule from entering the mouthpiece and hence the respiratory tract of the patient. The screen 20 is also shown in Figure 5.

Figure 2 also shows a cover 22 which can be opened by pivoting about a hinge line 24 for exposing the interior of the swirling chamber. The chamber comprises a generally cylindrical portion 26 having a tangentially arranged recess 28 for receiving a capsule to be pierced in the chamber. The piercing mechanism will be described later, with reference to Figure 3, but it is important to note that the present invention provides for a preferred positioning of the capsule-opening means as a pair of piercing pins operating inside the chamber 26,28 so that there is no need for the capsule to be inserted into the swirling chamber while its ends are open.

As explained above, in the relaxed configuration the limbs of the spring 10 are evident in the arcuate recesses 12, and Figure 2 shows that both limbs of the spring are substantially flush with the right-hand edges of the main body 4.

Figure 3 shows the left-hand limb of the spring 10 in this position but the right-hand limb 10 has been pressed inwardly so as to cause the sharp tip 30 of the capsule-piercing pin 32 carried by the depressed limb of the spring 10 to move inwardly of the recess portion 28 of the chamber to pierce the capsule there shown in chain-dotted lines. Also shown in chain-dotted lines, on the left-hand side of Figure 3, is the sharp tip of the opposite capsule-piercing pin in its capsule-piercing position.

As shown in dotted lines on the left-hand side of Figure 3, the capsule-piercing pin 32 is generally L-shaped and has a portion 34, shown in broken lines embedded in the limb of the spring while the substantially right-angle bend and the other limb of the spring project towards the recess portion 28 of the chamber. In order to guide the pins during their capsule-piercing movement, particularly bearing in mind the fact that the hemispherical domed end of the capsule will tend to deflect the sharpened tip 30 of the pins sideways as the two pins begin to press against the domed ends of the capsule, the two capsule-piercing pins are guided in respective locator bodies 36 which are positioned as close as possible to the position of the capsule in the recess 28 in order to ensure positive centring of the pins 32 during capsule piercing.

The right-hand side of Figure 3 shows that a stud 38 integrally formed with the limb of the spring 10 has moved inwardly of the body of the inhaler through a hole 50. By contrast, the stud 38 of the left-hand limb of the spring is clear of the space between two walls 40 of the inhaler body 4 and is retracted with its tip in the hole 50.

The purpose of the two studs 38 is to provide an interlock between the limbs of the spring 10 and the cover 22 which can be opened to allow access to the swirling chamber for permitting loading of a capsule in the swirling chamber recess ready for piercing.

As shown in Figure 5, the upper end 44 of the cover stands proud of the adjacent surface of the inhaler body in order to allow the user to place a thumb or a fingernail behind the upper end 44 to pull the cover 22 into the open or "second" position 22' partly shown in chain-dotted lines in Figure 5.

As also shown in chain-dotted lines, and in solid lines in its position occupied when the cover 22 is in its closed or "first" position, there is an optional hole 46 of a generally semi-circular ear 48 of the cover 22. Each side of the cover has a separate such ear 48, each provided with its respective hole 46, and the two holes 46 register with similar holes 50 in the wall portions 40 but only when the cover 22 is in its closed position shown in full lines in Figure 5. In all other positions of the cover 22, which is pivotable about the axis 24 (Figure 2) by virtue of pivot lugs 52 shown in chain-dotted lines in Figure 5, the ears 48 are immediately adjacent, and between, the walls 40 and close off the holes 50, thereby preventing the limbs of the U-shaped spring 10 from being squeezed together into the position shown on the right of Figure 3. Hence, because the studs 38 are unable to move towards one another, equally the sharpened tips 30 of the capsule-piercing pins 32 are located in the position shown on the left-hand side of Figure 3, i.e. fully retracted into the locators 36 where there can be no risk of inadvertent piercing of the finger of a user.

It is alternatively possible for the optional holes 46 to be omitted and for the ears 48 to be of different shape which will allow the operation of the capsule-piercing pins during positions other than the fully closed position of the cover 22, but the preferred arrangement shown in Figure 5 is considered the safest one where the optional holes 50 are provided and will be closed off at all times other than when the cover 22 is fully home.

The risk of such piercing of the user's finger can arise not only through deliberate misuse of the device by a child playing with it, or through accidental mis-use of the device by either an adult or a child, but also through normal manipulation of the device to clean the swirling chamber 26,28 of agglomerated powdered medicament resulting from previous uses of the inhaler. In view of the need to clean the device, it is an advantage to have the interlock to prevent the capsule-piercing pins from entering the recess 28 while there is access to the chamber.

The shape of the swirling chamber, with its generally cylindrical portion 26 and its generally tangential capsule-receiving recess 28 where the capsule-opening means are effective, is already disclosed in WO-A-91/19524 and is considered highly advantageous in resulting in both (i) centrifugal ejection of the contents through the swirling action of the vortical inhalation airflow entraining the capsule to cause it to tumble or spin within the chamber, and also (ii) percussive ejection as the ends of the capsule knock against the non-circular chamber sidewall, particularly at the obtuse corners between the recess 28 and the generally cylindrical chamber portion 26. It is also disclosed in WO-A-91/19524 that the generally flat nature of the chamber results in the capsule always being held in the same plane (the median plane of the chamber) in order to enhance the regularity and the intensity of the rotational tumbling of the capsule to eject the powdered medicament centrifugally.

Figure 3 shows the two air passages into the swirling chamber, one of them tangentially inwardly from the air inlet 14 also shown in Figure 1, and opening generally tangentially into the generally cylindrical chamber 26 at a port 54 from which the entering air stream will strike the capsule (shown in chain-dotted lines in Figure 3) to assist in ejecting the capsule from the capsule-rupturing recess 28 (bearing in mind that by now the two pin tips 30 will have been retracted into the locators 36) thereby liberating the capsule for entrainment in the vortical flow within the chamber 26.

The other air inlet 56 communicates with a passage to a port 58 discharging the inhaled air tangentially along a direction which is non-parallel with respect to the direction of air from the port 54 mentioned above.

As shown in Figure 3, the bight 60 of the U-shaped spring 10 is secured to the main body 4 of the inhaler by means of a hollow stud 62 also shown in Figure 5. In practice, the main body portion 4 consists of a front part 64 in which the mouthpiece 6 is formed, and a rear piece 66 which is bonded to the front piece 64, in this case by ultrasonic welding of the two parts 64,66 of the body portion 4 at their inter-engaging regions.

As an alternative, it is possible for the two parts 64,66 of the main inhaler body 4 to be secured together by a catch engagement means providing a snap fit which will facilitate assembly of the inhaler body. A detail of such a variation is shown in Figure 6 where the components which are also shown in Figure 5 are given the same reference numerals and the catch detent features 68 are also shown.

It will, of course, be understood that the grid screen 20 is embedded in the chamber-defining portion 72 from the time of its manufacture. Alternative methods exist. For example the grid may be insert-moulded into a containing ring which is then inserted into the mouthpiece.

In order to minimise the extent to which the released powdered medicament can agglomerate on the surface of the air passage through the inhaler, the chamber portion 72 is formed of a polymer of low surface resistivity, thereby having anti-static properties. Preferably the material defining the inside wall of the chamber 26,28 is a polymer having a surface resistivity of less than 10¹² Ohms or more preferably less than 10⁸ Ohms. In the present embodiment, the entire chamber defining body portion 72 is formed of the same low surface resistivity polymer, but if desired the chamber is provided with an inner lining of the low surface resistivity material.

The term "anti-static material" as defined herein is intended to denote a material which does not readily exert an electrostatic attraction for the powdered medicament released into the inhaler from the opened capsule. The antistatic properties may be derived by having its surface of a high static dissipativity on, and/or by having a high electrical conductivity, and/or by having a low surface resistivity.

There are various additives known to increase the anti-static properties of polymers, for example by increasing the electrical conductivity or reducing the surface resistivity, or enhancing the static dissipativity properties. One possibility is to incorporate carbon or steel filler, often in the form of fibres, into the polymer used for manufacture of those components to be given enhanced anti-static properties. This enhances the electrical conductivity and/or lowers the surface resistivity. Alternatively non-fibrous chemical additives, often blended into the moulding polymer in chip form prior to the moulding process, may be used to lower the surface resistivity in the moulded product. The product Pebax manufactured by the company Atochem of France is a polyether block amide product which may be obtained in an anti-static grade by use of such additives.

Another possibility is for the moulded component to be coated with an electrically conducting layer which thus reduces the surface resistivity.

Preferably the surface resistivity is less than 10¹² Ohms, and more preferably it is less than 10⁸ Ohms.

More preferably the mouthpiece 6 has its inner wall formed of such a material and more preferably still the cover 22 which closes the back of the swirling chamber is itself formed of such a polymer of low surface resistivity.

Figure 5 shows the optionally removable capsule reservoir box as comprising a box portion 74 and a lid 76 hingeably connected thereto by means of a thin film hinge 78. The box includes the sliding cover 16 mentioned above, and is held in place in the device by being a friction fit between the bottom surface 82 of the box and the engaging face of the front body portion 64 of the inhaler and also between the upper edge surface 184 of a domed portion 186 of the lid 76 and the abutting edge of the recess in the inhaler front body portion 64 which receives that domed cover portion 186.

The purpose of removing the capsule reservoir 74,76 is to allow the reservoir to be removed and kept dry while the rest of the device is being washed to clear any build-up of agglomerated powdered medicament from the walls of the inhalation air passage. In this way, the reservoir 74,76 which does not ever become exposed to the powdered medicament (because the capsules contained in the reservoir are always closed) can be kept apart from those parts of the inhaler which need to be washed in order to remove agglomerated medicament. Then, when the washed parts of the device have been thoroughly dried, ready for future use, the reservoir box can be reinserted.

Another advantage of being able to remove the reservoir is that this allows the pharmacist to dispense a package comprising the capsules already contained in a reservoir which can then simply plug into the inhaler in place of an empty reservoir which can either be disposed of or recycled.

If the reservoir 74,76 is to be reused, then replenishment of its supply of capsules is achieved by first of all removing the reservoir 74,76 from the rest of the inhaler 2 and then opening the lid 76 to expose the interior or the reservoir box 74. If, inadvertently, any of the capsules have become moist and the gelatine body of the capsule has begun to soften and become sticky, then such capsules can be readily removed when the lid 76 has been opened.

A fresh supply of capsules is then inserted in the box 74 and the lid 76 is then closed and the reservoir once more inserted into the inhaler 2.

As indicated above, the sliding cover 16 is part of the reservoir and can be drawn downwardly in order to liberate a top capsule, shown at C in Figure 5. For this purpose, the sliding cover 16 has a shoe 84 dimensioned so as to be received within a slot 86 of the box 74 and having a wider edge portion 88 which is able to be forced through the slot 86 but then prevents removal of the slide 16 from the box 74 by virtue of the edge portion 88 being too wide to return to the slot 86.

The sliding cover 16 is also provided with a detent shoe 90 which is able to engage over a bar 92 of the box 74 in the uppermost position of the slide but which can only pass over the bar 92 when the sliding cover 16 is pulled strongly downwardly so as to cause the slide to bend as the shoe 90 rides up over the bar 92. There will similarly be a catch engagement action holding the sliding cover in its uppermost position when the slide is again returned to the upper position. It will be appreciated that the upper and lower ends of the detent shoe 90 are chamfered in ordered to ease passage of the shoe 90 over the bar 92 in either direction of movement.

The alternative configuration illustrated in Figure 6 is intended simply to exemplify an alternative way of assembling the inhaler and it is not envisaged that in the life of the inhaler the catches 68 will ever need to be released. The provision of those catches is intended simply to facilitate the operation of assembling the inhaler after moulding.

In Figure 6, the grid 20 is shown insert-moulded in a mounting ring 70 and the mouthpiece 6 is integral with the swirling chamber-defining component. This integral component 6 is injection moulded of a suitable anti-static polymeric material.

The operation of assembling the inhaler 2 shown in the drawings is performed as follows:-

The grid 20, already insert-moulded in the mounting ring 70 (Figure 6), is first snapped in place (unless the grid is embedded in the chamber-defining portion 72 as in Figure 5, or in the mouthpiece.

The two retainers 36 for the piercing pins 32 will then be slid into place in the chamber into the positions shown in Figure 3. The integral mouthpiece and chamber component 6 is then inserted into the front cover 64.

The pins 32, 34 are inserted into the spring 10.

Then the U-shaped spring 10 and the front body portion 64 of the inhaler body are fastened together by insertion of the hollow stud 62 of the front body portion 64 into the socket provided in the bight of the spring 10. With the spring in the open position (as shown at the left-hand side of Figure 3) the cover 22 is then snapped in place with the pivot lugs 52 of the ears 48 inserted between the two wall portions 40 snapping into place in the holes 53 formed to receive them.

Then, with the cover 22 in the closed position, the rear body portion 66 is offered up to the rest of the assembly and united to it either by ultrasonic welding at the points of contact between the two portions 66 and the rest of the assembly or by catch-engagement as the portion 66 is pressed against the rest of the assembly so that the catches 68 ride over the corresponding ribs such as 94 in Figure 6 by virtue of deformation of the legs 96 on which the catches 68 are defined.

Finally, the mouthpiece cover 8 is snapped in place and the capsule reservoir 74,76 is itself forced into place. It will, of course, be understood that assembly of the sliding cover 16 to the box 74 is achieved simply by pressing the sliding cover firmly against (i.e. rightwardly as viewed in Figure 5) the box 74 to drive the detent edge 88 of the blade 84 through the slot 86.

Although, in the above description, the mesh screen 20 is described as a stainless steel mesh, which has the advantage of being electrically conductive, the mesh can be of any other suitable optionally anti-static material which may be electrically conductive, or electrostatically dissipative or of low surface resistivity, and which is non-corrosive and will resist washing. For example, it is possible to mould a mesh integrally with the rest of the chamber-defining portion 72 as an integral component.

All the components of the inhaler may be formed by injection moulding, and as will be seen the only complications are the need for embedding of the L-shaped pins 32,34 in the ends of the limbs of the spring 10, and the embedding of a mesh 20 in the chamber-defining portion 72.

The purpose of having separate retainers 36 for the support of the capsule-piercing pins 32 is to ensure that there will be no problems of trying to mould a passage (for the pin 32) having a bore which therefore closely matches the profile of the pin 32, along a direction which converges with the direction of the air passage between air inlet 14 and port 54 at the right-hand side of Figure 3. The retainers could also act as an additional seal to avoid the ingress of air into, or the loss of powder from, the inhalation chamber.

The use of the device shown in the drawings is as follows:-

Normally the device will be stored in a container, or even a pocket or handbag, with the mouthpiece cover 8 attached and all the capsules stored in the removable reservoir 74,76. When an inhalation is to be effected, the user slides the cover 16 of the reservoir 74,76 downwardly by pressure with the thumb on the thumb-grip ribs shown in Figure 2, to expose the slot normally occupied by the chamfered blade 90 of the sliding cover 16. This is best carried out with the device already inverted so that the capsule shown at C in Figure 5 is in position ready to leave through the slot once the sliding cover 16 has been retracted far enough.

After ejection of one capsule such as C, the sliding cover 16 is returned to the Figure 5 position to close off the reservoir 74,76 and then the cover 22 is opened either by catching a fingernail or the end of the thumb or finger behind the upper edge 44 and pulling the top of the cover 22 (as viewed in Figure 5) away from the main body 4, or possibly by downward pressure on the very bottom end of the cover 22 (although this latter movement may impose too much strain on the hinge lugs 52 and is not the most straightforward way of opening the cover 22). The capsule C can then be placed manually in the capsule-piercing recess 28 (Figure 3) ready to be pierced by the sharpened tips 30 of the pins 32. A capsule is shown in such a position in chain-dotted lines in both Figure 3 and Figure 5.

The cover 22 is then once again closed and snapped shut, and only now is it possible to operate the capsule-piercing mechanism, by virtue of the holes 46 in the ears 48 having come into register with the studs 38 on the spring 10. At this stage the mouthpiece cover 8 is removed and the mouthpiece is exposed ready for inhalation.

The user then squeezes the exposed areas of the limbs of the in-shaped spring 10 at the recesses 12 (Figure 2) to drive the pins 32 towards one another to pierce the ends of the capsule, guided by the locators 36. The capsule-piercing operation is concluded by releasing the limbs of the spring 10 to return to the Figure 2 position (see the left-hand side of Figure 3).

The user now places his or her lips over the mouthpiece 6 and inhales, thereby entraining an inward airflow through each of the inlets 14,56 (Figure 3) and into the chamber 26,28 by virtue of the ports 54,58 (Figure 3) and this airflow will generate a vortex inside the circular region 26 of the chamber which lifts the capsule from the position (shown in Figure 3) in the recess 28 and causes it to tumble rapidly in a spinning action in which the capsule has a transverse axis substantially coincident with the axis of symmetry of the cylindrical part 26 of the chamber. The fact that there are only two ports 54 and 58, together with the fact that they are not parallel to one another, and the existence of the capsule-piercing recess 28 tangentially with respect to the cylindrical chamber 26, will all help to create a percussive impacting of the capsule on the walls of the chamber 26,28 during its rotation, thereby ejecting the powdered medicament within the capsule through the pierced ends of the capsule both as a result of centrifugal action due to the spinning of the capsule and as a result of the percussive knocking of the capsule on the walls, and in particular on the corners of the chamber walls between the capsule-shaped recess 28 and the generally cylindrical remainder 26 of the swirling chamber.

If the ends of the capsule fracture during the piercing operation and cause brittle fragments to be released, these cannot enter the respiratory tract of the user because the inhaled air and entrained pulverulent medicament from the swirling chamber must first pass through the screen 20 before entering the mouthpiece 6. This screen 20 will thus hold back any fragments of capsule, and will certainly hold back the main portion of the capsule against swallowing by the user.

Where the capsule does disintegrate in this way, the device in accordance with the present invention is well able to deal with this problem because the capsule is retained inside the swirling chamber, behind the screen 20, during both capsule piercing and inhalation, whereas in other devices such as that of EP-A-0 333 334 the capsule is opened by pushing it against a fixed pin in the chamber before the chamber is closed, so any fragmentation of the capsule cap or capsule body will result in loss of the medicament, giving rise to an inadequate dose. In this earlier device, the problem is compounded because of the need to pierce both ends of the capsule separately, whereas in the present device, the two ends of the capsule are pierced simultaneously.

Hopefully, all the powdered medicament will pass into the respiratory tract of the user, and indeed it has been found that the device in accordance with the present invention achieves high rates of removal of the powdered medicament from the capsule, and high efficiency of removal of the medicament from the inhaler as a whole, while keeping the pressure drop across the inhaler at a minimum. This low drop in pressure across the device is particularly important, bearing in mind that the principal purpose of the device is to administer a medicament to exhibit therapeutic effects on the respiratory tract of a patient whose respiratory tract is therefore already rather weak and unable to generate a strong pressure differential.

For example, at an inhalation airflow of 60 litres per minute, the device has been measured to effect a 98% efficient emptying of the powder from the capsule shell and to present a pressure drop of 35.9 Kpa (26.9 cm water gauge) whereas at 30 litres per minute inhalation airflow, the efficiency of emptying the capsule has only dropped to 93%, and the pressure differential has dropped to 9.2 KPa (6.9 cm water gauge). The pressure drop at 40 litres per minute is 17 KPa (12.8 cm water gauge), that at 50 litres per minute it is 25 KPa (18.8 cm water gauge), and that at 20 litres per minute it is 3.7 KPa (2.8 cm water gauge). The fact that dropping the inhalation flow rate from 60 litres per minute to 30 litres per minute only causes a slight deterioration in the efficiency of emptying of the capsule is particularly significant because it means that those patients already having a seriously deficient respiratory function are well able to inhale a very high proportion of the total medicament available in the capsule on one inhalation.

By way of comparison the device was tested at the same flow rates, but without a capsule present in the swirling chamber and the pressure drops across the device were as shown in Table I.

**TABLE 1**

| | | | | |
|---|---|---|---|---|
| Flow rate (l/min) | 20 | 30 | 40 | 60 |
| Pressure drop in KPa (cm H₂0) | 4.5(3.4) | 10.7(8.0) | 19.9(14.9) | 43.9(32.9) |

Surprisingly it has been found that the pressure drop actually decreases when a capsule is present, thus aiding the inspiration by the patient.

By virtue of the low surface electrical resistivity of the appropriately selected material for the swirling chamber internal walls of the inhaler, and possibly also the mouthpiece, only a minimum of the powdered medicament remains on those walls to cause an agglomeration to build up with that resulting from subsequent uses.

However, it is nevertheless expedient to wash the air passageways from time to time and with the device illustrated this may be effected by firstly removing the capsule reservoir 74,76 where possible, and by then thoroughly washing the device, and drying it afterwards. Once the device is fully dry, the capsule reservoir 74,76 can be reinserted and the mouthpiece cover 8 reattached, to prepare the device for future storage and subsequent inhalations. It will, of course, be understood that the operations of washing and drying the swirling chamber and the mouthpiece are effected by opening the pivotal cover 22 on the back of the inhaler in order to allow access to the interior of the swirling chamber.

The device in accordance with the present invention has a further advantage in that the measured efficiencies of discharge of the powdered medicament from within the capsule are readily reproducible given the particular inhalation airflow rate applicable.

Additionally, because of the design of the chamber, the turbulent airflow generated, and the percussive action coupled with the centrifugal component of the capsule motion, the performance of the device remains fairly consistent over a wide range of inspiration rates. Table 2 shows the performance of the device.

Assessment of performance was carried out using an existing two-stage impinger, (apparatus A, described at pages A204 to A207 (Appendix XVII C) in the British Pharmacopoeia, 1988), which gives an indication of the quantity (or amount) of drug likely to reach the bronchioles and alveoli of the person using the inhaler according to the quantity of powder reaching the second stage of the impinger.

A model drug, Salbutamol sulphate was employed in the tests.

It can be seen that both delivery of drug to the lungs and reproducibility of delivery are very good and better than comparable commercial devices.

**TABLE 2**

| Performance of the Inhaler using a B.P. Two Stage Impinger (apparatus A). (a) Percentage of Dose reaching the second stage of the impinger. | |
|---|---|
| Airflow (l/min) | (%) |
| 20 | 22.1 |
| 30 | 28.5 |
| 60 | 25.9 |

| (b) Reproducibility of Dose delivery; 6 successive individual capsule actuations. Result expressed as relative standard deviation (percent). | |
|---|---|
| Airflow (l/min) | (%) |
| 20 | 13.8 |
| 30 | 4.3 |
| 60 | 5.4 |

Hence, it is considered that the use of the device is not subject to any degree of skill or practice on the part of the operator; provided the patient ensures that the capsule is in the correct position before operating the capsule-piercing pin by squeezing the spring 10, the capsule will always be open at both ends and will always liberate an extremely high proportion of its contents, even at low inspiration rates.

In the embodiment of Figures 1 to 5, the mouthpiece 6 may be integral with the chamber-defining component 72 as shown in Figure 6, and this would indeed be preferable as eliminating a parting line at which the powdered medicament could agglomerate.

Although cleaning may be by wiping clean or washing, the dry cleaning operation is preferred because the presence of residual moisture will cause even more problems than any static charge on the mouthpiece and chamber.

## Claims

1. An inhaler (2) for inhaling powdered medicament from capsules which are opened in the inhaler, comprising:-a swirling chamber (S) having air inlets and able to hold a capsule (C) from which the powdered medicament is extracted by rotation of the opened capsule in the inhalation airflow through the swirling chamber; means (30) in the swirling chamber for piercing a capsule having been inserted in the swirling chamber ready for inhalation; and a closure member (22) movable between a first position (22) closing the swirling chamber and a second position (22') providing access to the swirling chamber for insertion of a capsule, characterized by interlock means (38, 46, 48) responsive to positioning of the closure member in said second position for inhibiting operation of said capsule piercing means.

2. An inhaler according to claim 1, characterized in that said capsule piercing means comprise at least one pin (30) drivable for piercing the capsule to provide an opening through which the powdered medicament can be released during swirling.

3. An inhaler according to claim 2, characterized in that there are two of said pins (30) and they are mounted to approach one another colinearly for piercing the domed ends of an elongate capsule, and in that the swirling chamber includes a recess (28) to support the said elongate capsule during operation of said pins.

4. An inhaler according to any one of the preceding claims, characterized in that said closure member (22) is movable from said first position (22) to said second position (22') by pivoting.

5. An inhaler according to any one of claims 1 to 3, characterized in that said interlock means (38, 46, 48) are responsive to arrival of said closure member at said first position (22) for liberating the capsule-piercing means for operation while said closure member is in said first position, and are effective to inhibit operation of said capsule-piercing means (30), both in said second position (22') and in all intermediate positions.

6. An inhaler according to claim 5, wherein said closure member (22) is movable from said first position to said second position by pivoting, and characterized in that said closure member comprises a pair of ears (48) each having an aperture (46) for registration with a stud (38) of said interlock means whereby said stud (38) is unable to move in the direction corresponding to operation of said capsule-piercing means until said holes (46) in the ears are in register with said studs (38).

7. An inhaler according to claim 6, characterized in that said ears (48) are flexible and each include a respective pivot lug (52) for engagement with a pivot recess of the body of the rest of the inhaler, whereby said ears may be deflected either towards or away one another in order to permit entry of said pivot lugs into said pivot recesses by deformation of the ears during assembly of the inhaler, whereupon the ears then spring back to their relaxed configurations to hold the closure member connected to the rest of the inhaler body.

8. An inhaler according to any one of claims 1 to 3, characterized in that the chamber (S) is non-circular and has two opposed walls (S', 20) joined by at least one further wall (26, 28) of non-circular shape, and is able to allow the capsule to rotate freely about its transverse axis under the influence of the inhaled airflow but otherwise to impact against said at least one further wall in order to increase the likelihood of ejection of the medicament from within the capsule.

## Patentansprüche

1. Inhalator (2) zum Inhalieren eines pulverförmigen Medikaments aus Kapseln, die im Inhalator geöffnet werden, mit einer Wirbelkammer (S) mit Lufteinlässen, die eine Kapsel (C) halten kann, aus welcher das pulverförmige Medikament durch Rotation der geöffneten Kapsel im Inhalationsluftstrom durch die Wirbelkammer extrahiert wird; Mitteln (30) in der Wirbelkammer zum Aufstechen einer Kapsel, die in die Wirbelkammer inhalationsbereit eingefügt ist; und einem Verschlußelement (22), das zwischen einer ersten Position (22), in welcher es die Wirbelkammer verschließt, und einer zweiten Position (22'), in welcher es einen Zugang zur Wirbelkammer zum Einfugen einer Kapsel bietet, bewegbar ist, gekennzeichnet durch Verriegelungsmittel (38, 46, 48), die auf das Positionieren des Verschlußelements in der zweiten Position ansprechen, um eine Betätigung der Kapselaufstechmittel zu verhindern.

2. Inhalator nach Anspruch 1, dadurch gekennzeichnet, daß die Kapselaufstechmittel mindestens einen Stift (30) aufweisen, der zum Aufstechen der Kapsel antreibbar ist, um eine Öffnung zu schaffen, durch welche das pulverförmige Medikament während des Herumwirbelns freigesetzt werden kann.

3. Inhalator nach Anspruch 2, dadurch gekennzeichnet, daß zwei dieser Stifte (30) vorhanden sind und daß sie so angebracht sind, daß sie sich einander kolinear nähern, um die gewölbten Enden einer länglichen Kapsel aufzustechen, und daß die Wirbelkammer eine Ausnehmung (28) aufweist, um die längliche Kapsel während der Betätigung dieser Stifte abzustützen.

4. Inhalator nach einem der vorhergehenden Ansprüche, dadurch gekennzeichnet, daß das Verschlußelement (22) durch Verschwenken aus der ersten Position (22) in die zweite Position (22') bewegbar ist.

5. Inhalator nach einem der Ansprüche 1 bis 3, dadurch gekennzeichnet, daß die Verriegelungsmittel (38, 46, 48) auf die Ankunft des Verschlußelements in der ersten Position (22) reagieren, um die Kapselaufstechmittel zur Betätigung freizugeben, während das Verschlußmittel in der ersten Position ist, und die wirksam sind, um die Betätigung der Kapselaufstechmittel (30) sowohl in der zweiten Position (22') als auch in allen dazwischenliegenden Positionen zu verhindern.

6. Inhalator nach Anspruch 5, wobei das Verschlußelement (22) durch Verschwenken aus der ersten Position in die zweite Position bewegbar ist, und dadurch gekennzeichnet, daß das Verschlußelement ein Paar Ansätze (48) aufweist, die jeweils eine Öffnung (46) zum Fluchten mit einer Nase (38) der Verriegelungsmittel haben, wodurch die Nase (38) sich nicht in die der Betätigung des Kapselaufstechmittels entsprechenden Richtung bewegen kann, bis diese Löcher (46) in den Ansätzen mit den Nasen (38) fluchten.

7. Inhalator nach Anspruch 6, dadurch gekennzeichnet, daß die Ansätze (48) biegsam sind und jeweils einen entsprechenden Schwenkzapfen (52) zum Eingriff mit einer Schwenkausnehmung des Körpers des restlichen Inhalators aufweisen, wodurch die Ansätze entweder zueinander oder weg voneinander gebogen werden, um durch Verbiegen der Ansätze während des Zusammenbaus des Inhalators zu ermöglichen, daß die Schwenkzapfen in die Schwenkausnehmungen gelangen, wonach die Ansätze in ihre entspannte Form zurückfedern, um das Verschlußelement mit dem restlichen Inhalatorkörper verbunden zu halten.

8. Inhalator nach einem der Ansprüche 1 bis 3, dadurch gekennzeichnet, daß die Kammer (S) nicht-kreisförmig ist und zwei gegenüberliegende Wände (S', 20) hat, die durch mindestens eine weitere Wand (26, 28) von nicht-kreisförmiger Gestalt verbunden sind, und die es der Kapsel ermöglichen kann, frei um ihre Querachse unter dem Einfluß des inhalierten Luftstroms zu rotieren, jedoch ansonsten gegen die mindestens eine weitere Wand anzuschlagen, um die Wahrscheinlichkeit des Ausstoßens des Medikaments aus dem Inneren der Kapsel zu erhöhen.

## Revendications

1. Inhalateur (2) pour inhaler un médicament en poudre à partir de capsules qui sont ouvertes dans l'inhalateur, comprenant : une chambre de tourbillonnement (S) ayant des entrées d'air et capable de retenir une capsule (C) dont le médicament en poudre est extrait par rotation de la capsule ouverte dans le flux d'air d'inhalation à travers la chambre de tourbillonnement; un moyen (30) dans la chambre de tourbillonnement pour percer une capsule ayant été insérée dans la chambre de tourbillonnement prête pour inhalation, et un organe de fermeture (22) pouvant être déplacé entre une première position (22) fermant la chambre de tourbillonnement et une deuxième position (22') fournissant l'accès à la chambre de tourbillonnement pour insertion d'une capsule, caractérisé par des moyens de verrouillage (38, 46, 48) réagissant à la position de l'organe de fermeture dans ladite deuxième position pour empêcher l'actionnement dudit moyen de perçage de la capsule.

2. Inhalateur suivant la revendication 1, caractérisé en ce que ledit moyen de perçage de la capsule comprend au moins une broche (30) pouvant être entrainée pour percer la capsule afin de fournir une ouverture à travers laquelle le médicament en poudre peut être libéré durant le tourbillonnement.

3. Inhalateur suivant la revendication 2, caractérisé en ce qu'il y a deux desdites broches (30) et qu'elles sont montées de façon à se rapprocher l'une de l'autre de manière colinéaire pour percer les extrémités bombées d'une capsule oblongue et en ce que la chambre de tourbillonnement comprend une cavité (28) pour supporter ladite capsule oblongue durant l'actionnement desdites broches.

4. Inhalateur suivant l'une quelconque des revendications précédentes, caractérisé en ce que ledit organe de fermeture (22) peut se déplacer de ladite première position (22) vers ladite deuxième position (22') par pivotement.

5. Inhalateur suivant l'une quelconque des revendications 1 à 3, caractérisé en ce que lesdits moyens de verrouillage (38, 46, 48) réagissent à l'arrivée dudit organe de fermeture à ladite première position (22) pour libérer le moyen de perçage de la capsule pour permettre son actionnement pendant que ledit organe de fermeture est dans ladite première position, et sont efficaces pour empêcher l'actionnement dudit moyen de perçage de la capsule (30), à la fois dans ladite deuxième position (22') et dans toutes les positions intermédiaires.

6. Inhalateur suivant la revendication 5, dans lequel ledit organe de fermeture (22) peut se déplacer de ladite première position vers ladite deuxième position par pivotement et caractérisé en ce que ledit organe de fermeture comprend une paire d'oreilles (48) ayant chacune une ouverture (46) pour correspondance avec un goujon (38) desdits moyens de verrouillage, ledit goujon (38) étant incapable de se déplacer dans la direction correspondant à l'actionnement dudit moyen de perçage de la capsule tant que lesdits trous (46) dans les oreilles ne sont pas en correspondance avec lesdits goujons (38).

7. Inhalateur suivant la revendication 6, caractérisé en ce que lesdites oreilles (48) sont flexibles et comprennent chacune une saillie de pivotement (52) respective pour engagement dans une cavité de pivot du corps du reste de l'inhalateur, lesdites oreilles pouvant être déviées soit l'une vers l'autre, soit l'une loin de l'autre afin de permettre l'entrée desdites saillies de pivotement dans lesdites cavités de pivot par déformation des oreilles durant l'assemblage de l'inhalateur, suite à quoi les oreilles reviennent alors élastiquement dans leurs configurations de repos pour maintenir l'organe de fermeture connecté au reste du corps de l'inhalateur.

8. Inhalateur suivant l'une quelconque des revendications 1 à 3, caractérisé en ce que la chambre (S) est non circulaire et possède deux parois opposées (S', 20) reliées par au moins une autre paroi (26, 28) de forme non circulaire et est capable de permettre à la capsule de tourner librement autour de son axe transversal sous l'influence du flux d'air inhalé, mais pour le reste de battre contre ladite au moins une paroi supplémentaire afin d'augmenter la probabilité d'éjection du médicament hors de la capsule.
